# EUROPEAN PATENT APPLICATION

(11) **EP 4 275 595 A1**
(43) Date of publication of application: **15.11.2023**
(21) Application number: 22173373.6
(22) Date of filing: 13.05.2022
(51) Int. Cl.: A61B 5/11, A61B 5/00

(54) **FINGER DEXTERITY DEVICE**

(71) Applicant: The Provost, Fellows, Foundation Scholars, and The Other Members of Board, of The College of The Holy and Undivided Trinity of Queen Elizabeth, Dublin 2 (IE)
(72) Inventor: HAYDEN, Conor, Dublin (IE); MURRAY, Deirdre, Dublin (IE); GERAGHTY, Dermot, Dublin (IE)
(74) Representative: Barker Brettell LLP

(57) **Abstract**

A device for measuring finger dexterity, comprising a thumb portion configured to be releasably secured to a thumb, a finger portion configured to be releasably secured to an index finger, wherein the finger portion and the thumb portion are connected by a flexible connector; a first rotatable element; a rotation sensor configured to sense rotation of the rotatable element, a microprocessor, wherein the microprocessor is in communication with the rotation sensor, and a transmitter configured for wireless communication with a computing device, wherein the rotatable element is configured to rotate when the finger portion and thumb portion move relative to each other, and wherein the microprocessor is configured to generate rotation data in response to rotation of the rotatable element, wherein the transmitter is configured to transmit the rotation data to the computing device.

## Description

### Technical Field

The present invention relates to a device and method for measuring motor function. More specifically, the invention relates to a device and method for measuring motor function for neurological practice by a finger tapping test.

### Background to the Invention

Amyotrophic Lateral Sclerosis (ALS) (also known as Motor Neurone Disease (MND)) is a progressive and ultimately fatal neurodegenerative disease. The finger tapping test (FTT) is one of the most widely used measures of motor function in neurological practice. It involves tapping the index finger against the thumb rapidly while a clinician judges whether the movement is normal or abnormal by visually evaluating amplitude, frequency and accuracy. Performance grading has been utilised (for example) in the Movement Disorder Society Unified Parkinson's Disease Rating Scale (MDS - UPDRS) which incorporates a finger tapping task which rates participants on a 5-point scale. There are currently two main methods used to evaluate the FTT: tip of index finger to tip of thumb or tip of index finger to distal crease of thumb. The distal crease of the thumb method is generally considered the more sensitive measure. However, visual grading is subjective and insensitive to small but meaningful changes.

Additionally, some known methods involve mobile applications; for example, the participant taps a touchscreen of a computing device with the appropriate finger (sometimes with the hand placed flat on a surface) as high and as quickly as possible. Most, if not all, of the known mobile applications of this type are not validated for clinical use and focus on sports and rehabilitation, but some include different tests such as a two-target finger tapping test. A known digital platform for Parkinson disease patients has been developed by Apptomics. Originally known as iMotor (Apptomics Inc., Wellesley Hills, MA, USA), it is a mobile application that contains a two-target finger tapping test in which participants had to alternatingly tap the centre of two circles on a touchscreen with their index finger as fast and as accurately as possible.

Existing devices to measure and/or quantify dexterity using the FTT include that described in WO2016031348A1 to quantify bradykinesia scores in Parkinson's Disease. The device contains two magnetic coils (one detection, one generation of magnetic field) placed on the index finger and thumb. The distance between the two coils corresponds to a voltage due to electromagnetic induction. The voltage is then converted into a value by a nonlinear calibration model. Metrics such as maximum amplitude, total distance vs. frequency of taps and standard deviation of local max./min velocities in the opening/closing motion are extracted from the finger tapping motion. The device was tested on Parkinson Disease patients, whose data was then normalized according to age.

However, known devices have limitations relating to data processing, data output and ease of use. It is an aim of the present invention to address, or at least mitigate, drawbacks of the prior art.

### Summary of the Invention

According to a first aspect of the invention, there is provided a device for measuring finger dexterity, comprising a thumb portion configured to be releasably secured to a thumb, a finger portion configured to be releasably secured to an index finger, wherein the finger portion and the thumb portion are connected by a flexible connector; a first rotatable element; a rotation sensor configured to sense rotation of the rotatable element, a microprocessor, wherein the microprocessor is in communication with the rotation sensor, and a transmitter configured for wireless communication with a computing device, wherein the rotatable element is configured to rotate when the finger portion and thumb portion move relative to each other, and wherein the microprocessor is configured to generate rotation data in response to rotation of the rotatable element, wherein the transmitter is configured to transmit the rotation data to the computing device.

The device facilitates digitisation of the FTT for use in neurological assessments by virtue of an advantageous measurement method. Unlike orientation sensors (accelerometer, gyroscope etc.), no filtering of the data is required - the data is clean and is immediately available for processing. The small footprint of the device also means that it is suitable for remote monitoring and is not hindered by a laborious set up procedure. No expertise is required for the operation of the device. The data is stored after every test completion based on input from the user or tester. The data storage process may also be automated regardless of input from the user or tester. The easy set-up also reduces error in the results - unlike IMU sensors, a small change in position or orientation will not affect the data output. The device can be zeroed before every test. The microcontroller and wireless capability remove any dependency on laptops and wired connections.

Preferably, the rotatable element is a magnet and wherein the rotation sensor is a magnetic rotary encoder. The inclusion of the magnetic rotary encoder means that there is no noise inherent in the device.

Preferably, one end of the flexible connector is affixed to the finger portion and the other end of the flexible connector is affixed to the thumb portion, and wherein the flexible connector is configured to wind around a second rotatable element when the thumb portion and finger portion move towards each other. A spiral torsion spring is optionally fixed to the second rotatable element, wherein the spiral torsion spring is arranged to contract when the thumb portion and the finger portion are moved away from each other. Optionally, contraction of the spiral torsion spring causes rotation of the magnet.

Preferably, the spiral torsion spring is housed in the thumb portion. Preferably, the microprocessor and transmitter are housed in a wrist portion, wherein the wrist portion comprises securing means for securing the wrist portion to a wrist.

The device preferably further comprises a timer configured to record the time required to complete a predetermined number of finger taps by a participant performing a finger tapping test.

According to a second aspect of the invention, there is provided a system comprising the device described herein and a computing device configured to receive time and rotation data, plot a graph of rotation data as a function of time and output the graph on a display of the computing device. Optionally, the graph is a plot of distance as a function of time.

The computing device is preferably configured to convert the magnet rotation data into distance data by division by a predetermined calibration factor. The computing device is further preferably configured to store data relating to the maximum finger extension of a participant. The computing device is optionally further configured to upload raw and/or processed test data to cloud storage.

The computing device may be further configured to calculate a score for each finger tapping test performed, wherein the score is a normalised height value divided by a normalised time value. The normalised height is preferably an average maximum extension distance between thumb and index finger recorded during a test divided by a maximum possible extension between thumb and index finger for a particular participant, and the normalised time is preferably the time to complete 10 finger taps divided by the fastest possible time to complete 10 finger taps. Preferably, the computing device is configured to upload raw and/or processed test data to cloud storage.

According to a third aspect of the invention, there is provided a method of measuring finger dexterity, comprising generating, by a device described herein, time and rotation data pertaining to a finger tapping test, wirelessly transmitting the time and rotation data to a computing device, generating, by the computing device, a graph of rotation data as a function of time and outputting, by the computing device, the graph on a display.

Optionally, the method further comprises calculating, by the computing device, a score value for a finger tapping test using standardised data. Preferably, the method further comprises analysing the data, by the computing device, to determine at least one of average extension height, maximum extension height, time taken to complete a predetermined number of finger taps, number of hesitations and time taken to complete one tap motion.

According to a further aspect of the invention, there is provided a method of generating data relating to finger dexterity, comprising receiving time and rotation data from a finger dexterity device, wherein the time and rotation data relates to a finger tapping test performed using the finger dexterity device, converting the rotation data to distance data by multiplying by a calibration factor, retrieving a stored distance value and the stored time value, and calculating a score value based on the time data and distance data and retrieved stored time and stored distance values. The rotation data may be magnet rotation data.

Preferably, the stored distance value is the maximum finger extension of the participant who performed the finger tapping test. The stored time value may be fastest known time to complete the finger tapping test.

The method may further comprise generating a graph of distance as a function of time and displaying the graph on a display. Preferably, the finger dexterity device is a device described herein.

Also provided is a computer readable medium storing executable instructions which, when executed by a processor, cause the method as described herein to be performed.

### Brief description of the Figures

The invention will be described with reference to the following figures in which:
**Figures 1a and 1b** show the main components of a device according to an embodiment of the invention;
**Figures 2a and 2b** show opposing views of a device according to an embodiment of the invention attached to a thumb and forefinger;
**Figure 3** is an isometric view of a component of the device according to an embodiment of the invention;
**Figure 4** is an exploded view of the component of Figure 3;
**Figure 5** is an exploded view of a component of a device according to an embodiment of the invention;
**Figure 6a and 6b** are exploded and isometric views of a component of the device according to an embodiment of the invention;
**Figures 7a and 7b** are isometric views of a sub-component of the device according to an embodiment of the invention;
**Figure 8a and 8b** are exploded and isometric views of a sub-component of the device according to an embodiment of the invention;
**Figures 9a and 9b** are isometric views of a sub-component of the device of the device according to an embodiment of the invention;
**Figure 10a** is a plan view of a sub-component of the device according to an embodiment of the invention;
**Figure 11** shows a PCB according to an embodiment of the invention;
**Figure 12** is a flow diagram of a method of generating finger dexterity data according to an embodiment of the invention;
**Figure 13** is a graph of exemplary processed data generated by a device according to an embodiment of the invention;
**Figure 14** is a graph of processed data generated by a device according to an embodiment of the invention;
**Figure 15** is a graph of processed data generated by a device according to an embodiment of the invention;
**Figure 16** is a graph of processed data generated by a device according to an embodiment of the invention;
**Figure 17** is a graph of processed data generated by a device according to an embodiment of the invention; and
**Figure 18** is a graph of processed data generated by a device according to an embodiment of the invention.

### Detailed Description

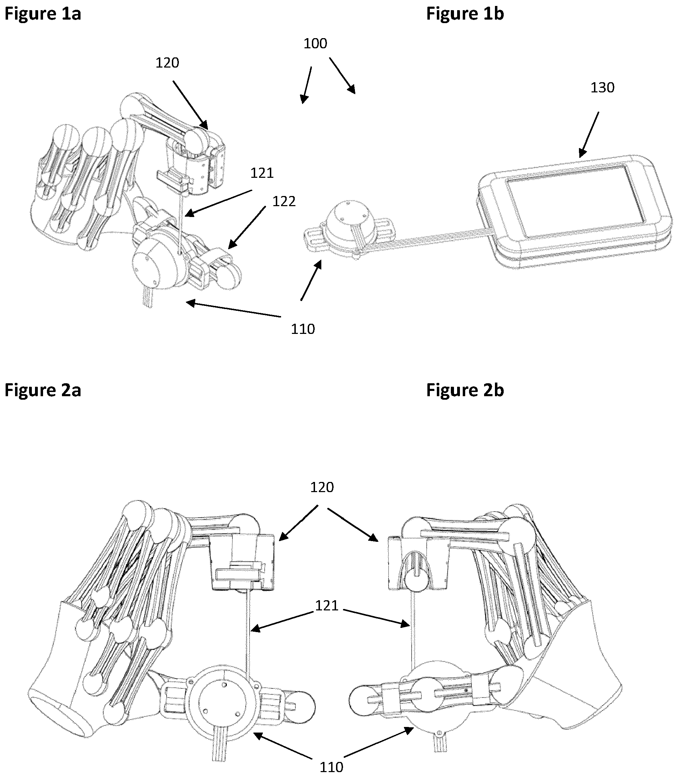

A finger dexterity device which digitises a finger tapping test is shown generally in Figures 1a and 1b. Device 100 comprises main body 110 which can be releasably secured to a participant's thumb with straps 122, which are preferably magnetic silicone straps. Straps 122 are adapted to enable attachment of many different thumb sizes and shapes. Finger attachment 120 is formed for attachment to the distal end of an index finger. As shown in Figure 1b, main body 110 is physically connected to module 130.

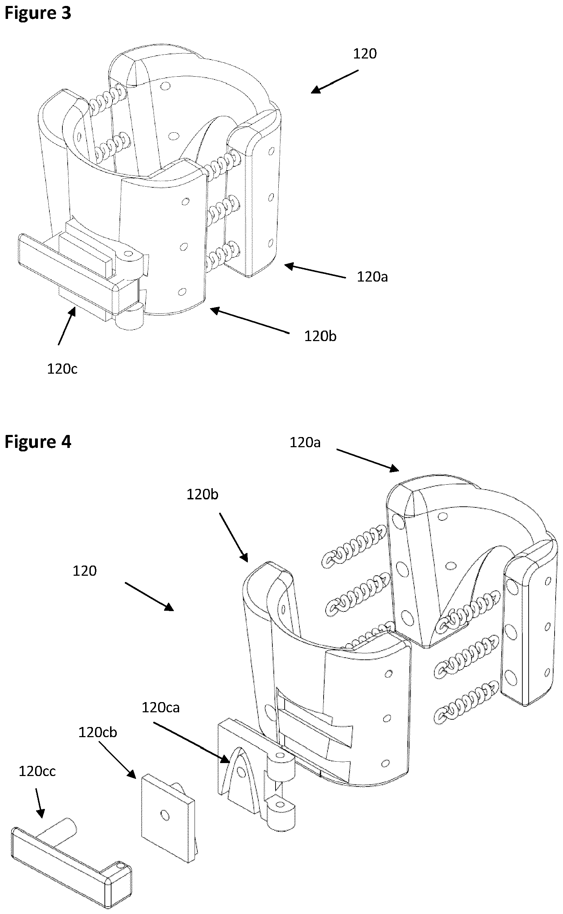

Figure 2a and 2b further illustrate the arrangement of the device components affixed to a thumb and index finger of a right hand. Extension member 121 connects the main body 110 to finger attachment 120. As will be described below, the visible length of extension member 121 varies as a participant's index finger moves up and down (i.e., away from, and back towards, their thumb). The lengthening and shortening of extension member 121 causes rotation of a magnetic rotary encoder located within main body portion 110. As shown in Figures 3 and 4, finger attachment 120 can be releasably secured to a participant's index finger, on the same hand on which the main body 110 is secured to the thumb. Finger attachment 120 is generally a two-part, spring-loaded component having an internal diameter than can expand to fit around an index finger and contract for securement. The spring-loaded mechanism allows finger attachment 120 to fit many different sized fingers.

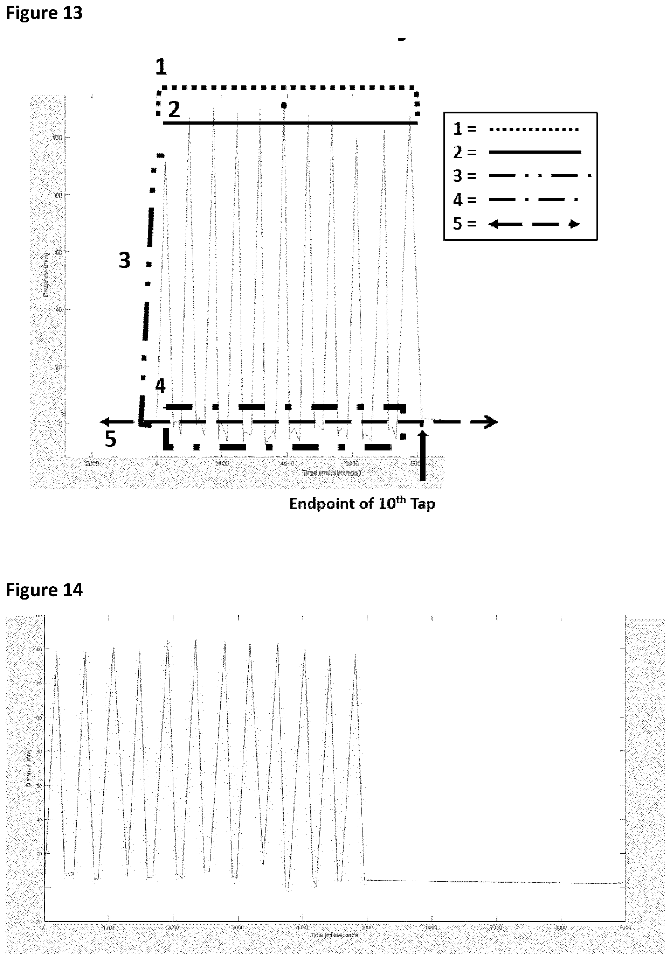

With reference to figure 4, finger attachment 120 generally comprises two opposing parts 120a, 120b connected by tension springs. Part 120a comprises a cut-out to allow a user to see their finger as they perform the test. Portion 120c (as shown in Figure 13) extends outwardly from portion 120b and provides means of detachable attachment of extension member 121. Portion 120c comprises at least one protrusion which fits within at least one groove on the outer surface of part 120b to create a dovetail joint. An end of extension member 121 fits into part 120ca and is secured in place by interference fit of part 120cb. A cylindrical protrusion on part 120cc is inserted into a corresponding hole or recess in part 120b to secure part 120c to part 120b.

As will be appreciated, parts 120a and 120b can be detached from part 120c whilst an end of extension member 121 is secured to portion 120c. As such, portions 120a and 120b can be changed (by, for example, replacement with similar parts with larger or smaller dimensions) to allow for securement to different fingers of differing sizes. Varying versions of portions 120a and 120b may have differing spring tensions to allow for greater or lesser extension and tension.

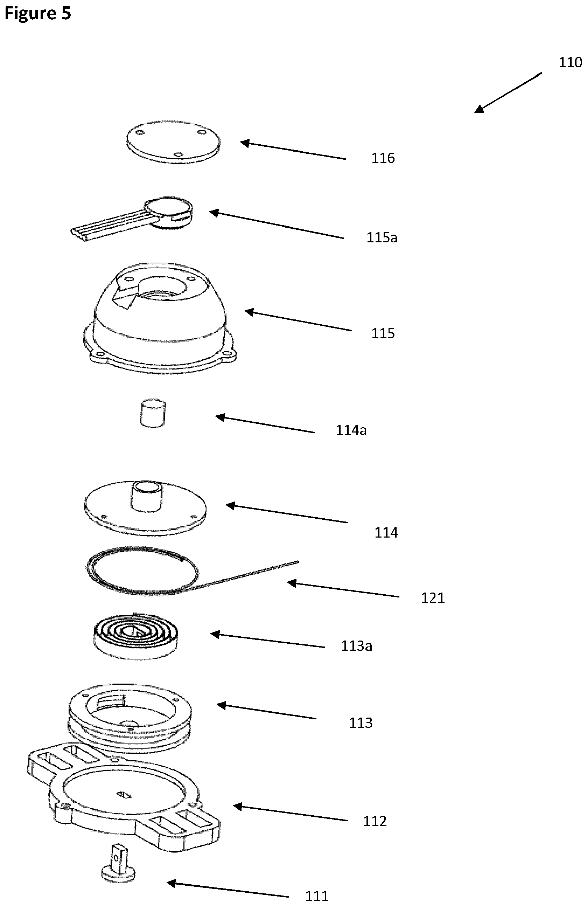

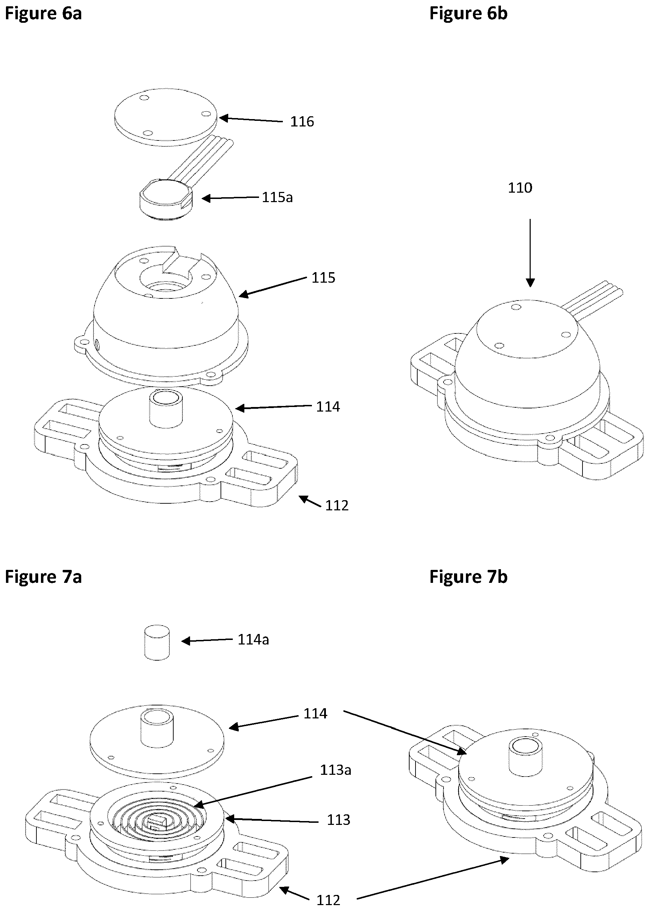

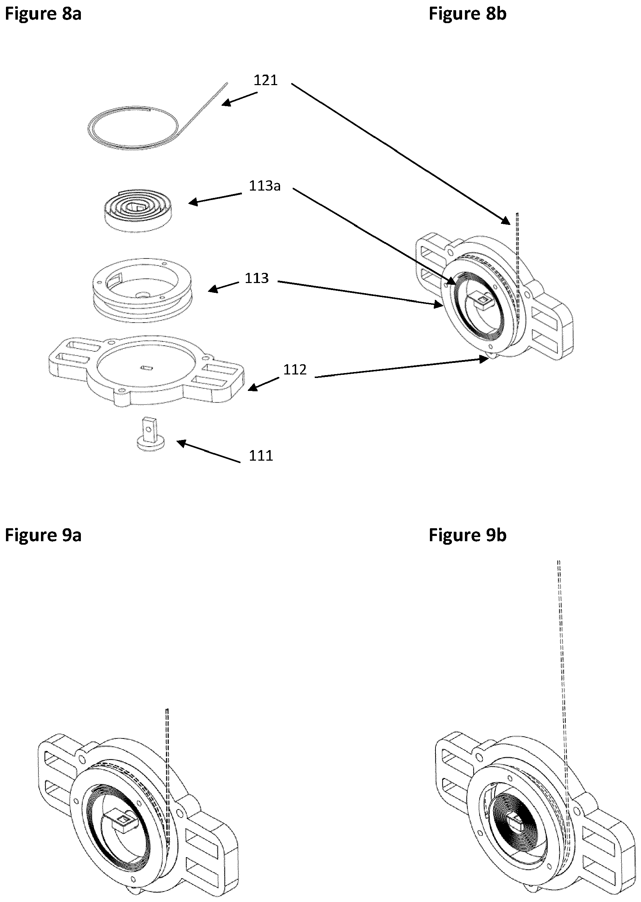

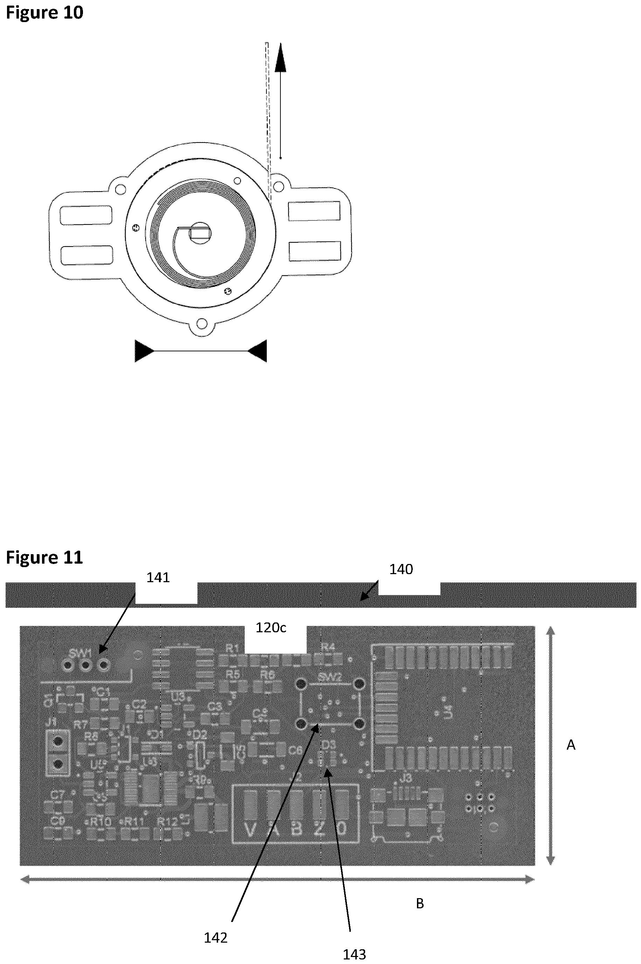

Main body portion 110 comprises multiple components, as shown generally in Figures 5, 6a and 6b.Base pin 111 fits within base 112. Base 112 comprises two opposing, laterally extending wings to facilitate attachment by attachment straps 122 and comprises a circular recess which is configured to receive and allow rotation of middle part 113 relative to base 112. Middle part 113 contains a hollow recess for receiving clock mainspring 113a. Clock mainspring 113a is affixed to middle part 113 such that clockwise rotation of middle part 113 causes clock mainspring 113a to wind/contract, and release/unwinding of the clock mainspring 113 causes counter clockwise rotation of middle part 113 (and thereby winding of extension member 121 around middle part 113). As illustrated in Figures 9a, 9b and 10, clock mainspring 113a contracts as middle part 113 rotates in a clockwise direction and reverts to its original position when middle part 113 rotates counterclockwise. Movement of the index finger away from the thumb causes extension member 121 to lengthen and unwind from the outer circumferential groove of middle part 113. This causes clock mainspring 113a to wind. As the participant moves their index finger towards their thumb, the tension in clock mainspring 113a is released, thereby causing extension member 121 to wind around the outer circumferential groove of middle part 113. The tension in clock mainspring 113a therefore ensures that extension member 121 winds around middle part 113 as the finger returns to the distal crease.

Container part 114 comprises a central cylindrical portion which is configured to house a 4x4mm cylindrical magnet 114a. Container part 114 is affixed to middle part 113 such that rotation of middle part 113 causes rotation of magnet 114a. Cover 115 fits over middle part 113 and container part 114 and is attached to base 112 with grub screws. Cover 115 comprises a hollow central chamber within which magnetic rotary encoder 115a is located. Lid 116 fits on the top of the cover 115 to secure magnetic rotary encoder 115a within cover 115.

In use, cover 115 and base 112 remain stationary as middle part 113 and container part 114 (within which magnet 114a is fixed) freely rotate clockwise and counterclockwise. Winding and unwinding of the clock mainspring causes clockwise and counterclockwise rotation of magnet 114a. The magnetic rotary encoder 115a detects rotation of magnet 114a located in container part 114 and 'counts' revolutions of magnet 114a. Pulses per revolution (ppr) refers to the number of periods on one of the quadrature signals in one revolution. Counts per revolution (cpr) is the number of changes of state on both channels of the encoder in one revolution and is achieved by electronically multiplying by 4, using the rising and falling edges of both channels of an encoder, i.e. cpr = 4 x ppr. The number of 'counts' per revolution depends on the resolution of the encoder - for example, a 5-bit encoder will record 32 pulses per revolution but will return a counts per revolution value of 8, i.e.; one revolution of the encoder = 8 counts. Similarly, a 12-bit encoder will record 4096 pulses per revolution and 1024 counts per revolution.

Magnetic rotary encoder 115a within main body portion 110 is connected to, and in communication with, module 130, as shown in Figure 1b. Module 130 is attached to a participant's wrist in use. Alternatively, module 130 can be placed in an appropriate location during the test that it does not interfere with the test or tester. Module 130 comprises a printed circuit board (PCB) on which a microcontroller, Bluetooth transceiver and rechargeable battery are fixed.

With reference to Figure 11, PCB 140 comprises connectors for on/off switch in location 141, push button in location 142 and LED in location 143. An LED is lit to denote whether magnetic rotary encoder 115a is counting (i.e whether a test is in progress). Width A is approximately 23mm and length B is approximately 53mm.

Module 130 is configured for selective wireless communication with a software application running on an external user device such as a smartphone, tablet or personal computer. The software application provides default control of device 100 via user interface features of the device in conjunction with the software application features. The test is preferably initiated from the software application. Initiation of a test begins a corresponding timer count in milliseconds. The counting is performed by a timer function of the microcontroller. In an alternative embodiment, counting is performed by a timer function of the software application, whereby the counting continues until the test is stopped via user input to the software application. A push button on module 130 may also be configured to act as a 'hard' stop for the counter in the event that the accompanying software used with device 100 fails to work. An alternative method for starting the test includes the push button on module 130. This may be used to 'zero' the test, such that when it is pushed, the magnetic encoder count is reset to zero.. A corresponding timer also begins when the push button is depressed. The counts per revolution recorded by magnetic encoder 115a correspond to the number of revolutions of magnet 114a in a clockwise (or counterclockwise) direction, as discussed above.

Extension member 121 is wound around middle part 113 and an end of extension member 121 is secured to middle part 113 to . As a participant's thumb and finger move apart, extension member 121 is pulled out of the casing by unwinding from middle part 113. This causes mainspring 113a to wind/contract. In some embodiments, extension member 121 is elastic. Container part 114 rotates with middle part 113 such that when extension member 121 is extends and unwinds from middle part 113, magnet 114a also rotates (in a clockwise direction (or counterclockwise direction, depending on the configuration). This rotation is recorded by magnetic encoder 115a inserted in component 115. Magnetic encoder 115a 'counts' a predetermined number per complete revolution; the number of counts per revolution being dependent on the resolution of encoder 115a. In later processing, the number of counts allows the number of revolutions to be determined, which in turn allows for the distance moved to be determined (i.e. the distance moved by the index finger away from the thumb crease).

As the participant draws their index finger back towards their thumb crease, extension member 121 slackens, allowing mainspring 113a to release/unwind back to (or closer to) its original or starting tension. This causes middle part 113 to rotate counterclockwise which in turn causes magnet 114a to rotate counterclockwise. Encoder 115a records the number of counts as magnet 114a rotates counterclockwise. Encoder 115a records the count value at each turning point, i.e. when a change in direction is recorded. In later processing, the number of counts recorded during rotation in a counterclockwise direction by magnet 114a is used to determine the number of revolutions, and thereby the distance moved by the index finger, relative to the thumb crease, as it travels back towards the thumb crease. The clockwise and counterclockwise count data is recorded by the microcontroller, along with the corresponding time in milliseconds for each turning point. These turning points are then summed to get the total duration of the test.

The duration of the test and the clockwise and counterclockwise count number from the encoder are output by the microcontroller to the Bluetooth transmitter, which is preconfigured (i.e. configured before the start of the test) to transmit the time and count data to the external computing device for processing and storage. The ability to wireless transmit data via the Bluetooth transmitter advantageously means that the only wires required for the device to operate are those from the encoder within main body 120 to module 130. The data is preferably processed by the software application running on the external computing device. In a preferred embodiment, the data is sent by the computing device to a cloud server for pseudonymised or anonymised storage. The collection and storage of data from multiple test data for multiple participants allows for normalisation of the data, as described further below.

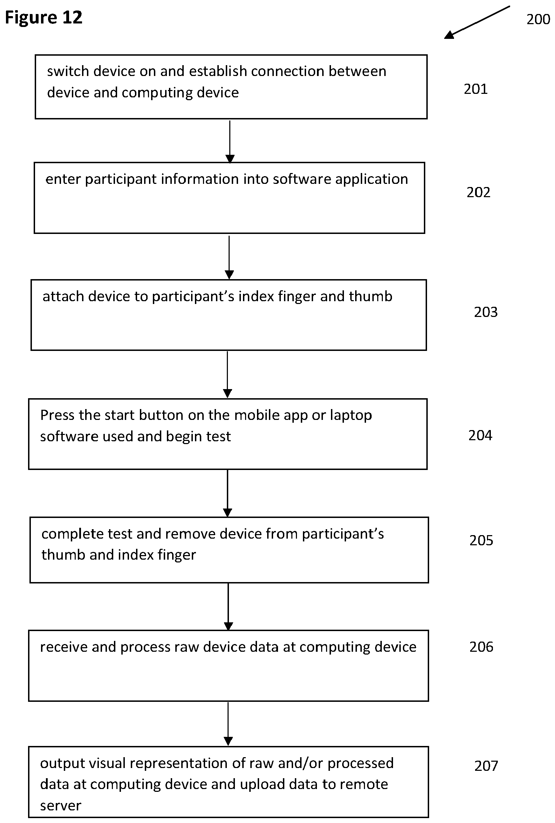

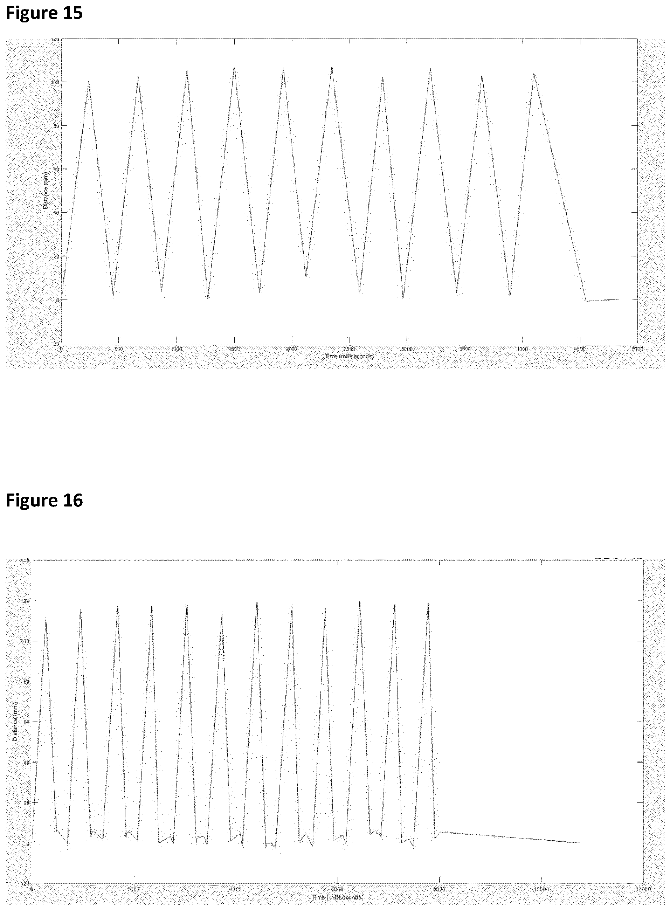

A process 200 of performing a test to generate the data is described with reference to Figure 12. At step 201, device 100 is switched on such that power from the battery is supplied to the components on the PCB and a Bluetooth connection is established between device 100 and an external computing device, according to mechanisms known in the art. At step 202, relevant information pertaining to the participant who will be performing the FTT is entered into a software application running on the computing device. For example, the participant may be a patient under clinical care, and may have a unique identification code. Preferably, age, gender, dominant hand and left/right hand test information is entered. At steps 203, 204 and 205, device 100 is attached to the participant's hand and the FTT test is performed. At step 206, the device data (i.e., the duration of the test and the counts for the clockwise and counterclockwise rotation phases) is transmitted to the computing device and processed to output, at step 207, a graphical representation of the test, as exemplarily shown in Figure 16. It will be appreciated that the order of the steps as outlined in Figure 15 depending on the particular implementation. Some steps may also overlap. For example, processing of the data at the computing device may be initiated upon receipt of the data from device 100 but whilst the device is still attached to a participant's finger. As a further example, a graphical representation of test data may be output after completion of one test whilst the device is attached to a participant's hand and before further test are started.

**Table 1**

| 1 | PhaseDuration | PulseCount |
|---|---|---|
| 2 | 0 | 0 |
| 3 | 33 | 0 |
| 4 | 0 | 1 |
| 5 | 218 | 1874 |
| 6 | 356 | 115 |
| 7 | 171 | 1721 |
| 8 | 325 | 77 |
| 9 | 183 | 1723 |
| 10 | 321 | 59 |
| 11 | 171 | 1753 |
| 12 | 371 | 62 |
| 13 | 162 | 1665 |
| 14 | 332 | 27 |
| 15 | 179 | 1672 |
| 16 | 115 | 90 |
| 17 | 183 | 89 |
| 18 | 33 | 74 |
| 19 | 170 | 1676 |
| 20 | 109 | 59 |
| 21 | 205 | 58 |
| 22 | 22 | 84 |
| 23 | 175 | 1744 |
| 24 | 333 | 46 |
| 25 | 170 | 1724 |
| 26 | 121 | 44 |
| 27 | 197 | 44 |
| 28 | 31 | 51 |
| 29 | 158 | 1685 |
| 30 | 104 | 47 |
| 31 | 559 | 47 |
| 32 | 18 | 47 |
| 33 | 0 | 0 |
| 34 | 0 | 0 |

Table 1 is an exemplary raw data set output by the microcontroller. '*Phase Duration*' is the duration of each phase is milliseconds, where a 'phase' is the magnet rotating in a particular direction; '*Pulse Count*' is the number of rotations of the magnet during a particular phase. For example, the 4^{th} phase (i.e., the 4^{th} change of direction) lasted for 218ms and the encoder counted 1874 ('pulses').

The magnetic rotary encoder is preferably 12 bit, such that one full revolution of the magnet is equal to 1024 counts per revolution. The counts are recorded for the duration the magnet is in a particular state, i.e., rotating in a particular direction. The count is reset to zero and counting beings again by the encoder when the magnet changes state, i.e., it switches rotation direction. The interval between each of change of state of the magnet is recorded in milliseconds. This is seen in the corresponding pulse duration value for every pulse count. It can be seen from Table 1 that there are 10 'peaks' in the pulse count column. These peaks correspond to maximum index finger extension away from the thumb crease.

The pulse count values are converted to millimetres by dividing the count value by a calibration factor which is dependent upon each specific device. This calibration factor is calculated using a static testing machine or any other controlled displacement test (e.g., Zwick Instron etc). For example, a static machine extends the flexible member by a known amount. The distance is referenced against the pulse count value output from the device. A number of different measurements are taken a set number of times to determine the calibration value - i.e., what 1mm displacement corresponds to in pulse count value. For example, 1mm = 18.02 counts. A raw data graph (for example, as shown in Figure 16) is generated by summing the phase durations (x-axis) and plotting corresponding millimetre value based on conversion of the pulse count by the calibration factor (y-axis). Figure 16 is an exemplary distance/time output of a finger tapping test using the device described herein. The y-axis shows the distance between the thumb crease and the index finger, and the x-axis shows the time in milliseconds.

The average extension height, max extension height and time to complete the test (for example, the time take to make 10 taps by the index finger at the thumb crease) can be determined from section 1 of the graph of Figure 13. The total number of extensions can be seen from section 2 of the graph, as well as the maximum distance of each extension. Section 4 of the graph (bottom rectangle) denotes the number of hesitations/time for one tap motion. The speed of each tap can be deduced from section 3 and the accuracy of each tap (i.e. how far the tap is away from the calibrated '0' position) can be deduced from a comparison with the horizontal line denoted by 5 on the graph.

Plotting the raw data distance/time as per the example of Figure 13 provides near-instant, useful information which can be reviewed by a clinician to identify differences or changes compared to calibrated or previous participant test data. Such differences are easily recognisable from a comparison of Figures 14, 15 and 16. Figure 14 is an exemplary test output by a 20-year-old male on the right hand. Figure 15 is an exemplary output by a 40-year-old female on the left hand, and Figure 16 is a 70-year-old male on the right hand.

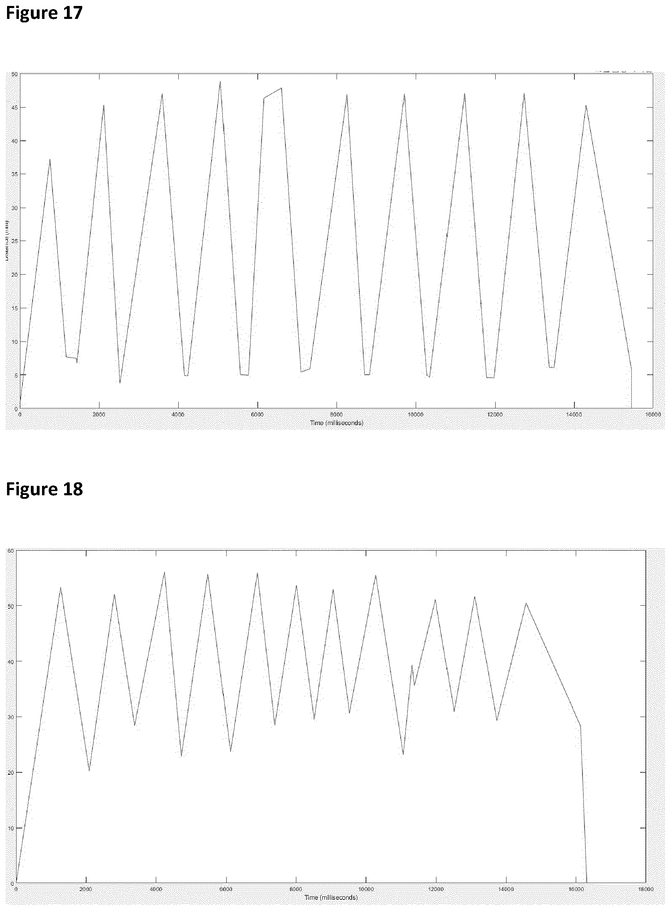

Most participants have a dominant hand (handedness) which is generally used for the fine motor tasks. The differences in test output by the dominant vs non-dominant hand can be seen from a comparison of Figures 17 and 18. Figure 17 is a test output of participant 1 using the left, dominant hand. Figure 18 is a test output of participant 2 also using the left hand, but the left hand is non-dominant.

In preferred embodiments, the computing device not only outputs the raw test data in graphical form, but also calculates and outputs an overall score for each test, as well as outputting values of average extension height, max extension height, time to complete the test, number of hesitations (and associated hesitation characteristics within each tap or over the test),time for one tap motion, speed of each tap, the accuracy of each tap and participant fatigue. Evaluation can contain combined parameters i.e., accuracy combined with speed, may be a meaningful data point. Any potential combination of factors may be useful. Normalisation techniques may be used on these values. A participant may perform a test multiple times (e.g., three times) to provide more accurate and statistically relevant data for clinical use. A 'passive' test (used to normalised for hand size) is performed on a participant's left and right hand to establish their maximum possible extension. This test is performed physically by the tester and recorded by the device as a separate data set. If one of the participant's tests is chosen as a best test for each hand, then a score is used to identify the test that should be used for such clinical or statistical purposes.

### Dominant Hand

**Table 2**

| **No.** | **AEH** | **TTC** | **PH** | **Normalised Height** | **Normalise Time** | **Overall Score** |
|---|---|---|---|---|---|---|
| 013R1 | 113.86 | 8148.00 | 130.41 | 0.87 | 1.23 | 0.71 |
| 013R2 | 110.40 | 7111.00 | 130.41 | 0.85 | 1.07 | 0.79 |
| **013R3** | **117.05** | **6638.00** | **130.41** | **0.90** | **1.00** | **0.90** |

### Non-Dominant

**Table 3**

| **No.** | **AEH** | **TTC** | **PH** | **Normalised Height** | **Normalise Time** | **Overall Score** |
|---|---|---|---|---|---|---|
| 013L1 | 103.16 | 7556.00 | 121.81 | 0.85 | 1.03 | 0.82 |
| **013L2** | **111.13** | **7329.00** | **121.81** | **0.91** | **1.00** | **0.91** |
| 013L3 | 109.03 | 7694.00 | 121.81 | 0.90 | 1.05 | 0.85 |

Tables 2 and 3 show relevant metrics for a healthy participant's right and left hand, where:
*AEH = Average Extension Height* (this is calculated by averaging the 10 peaks/extensions that correspond to a finger extension between thumb and index finger in a test)
*TTC = Time to Completion* (time in milliseconds to complete 10 taps)
*PH = Passive Height* (distance recorded when a tester/clinician extends the participant's thumb away from the index finger as far as possible)
*Normalised Height* (this is the AEH/PH and has a maximum possible value of 1. This can be converted to a percentage to show in real time what percentage of the passive height is reached)
*Normalised Time* (this is found by using the fastest TTC as the base level and dividing the 3 TTCs by this fasted TTC. The fastest time will always have a normalised time of 1. E.g., is the fastest TC is 6638, the normalised time in first row of table two = 8148/6638 = 1.23)

The overall score for the number of tests carried out for each hand per data collection set is calculated by dividing the normalised height by the normalised time. This is based on the test procedure whereby the participant extends their finger as high as they can, as fast as they can for 10 complete taps. The highest overall score can be manually or automatically identified and stored as pseudonymised or anonymised data for further statistical analysis, and/or used to identify the test to be used to make a clinical assessment. This overall score does not have to be used to find which of the tests is best. The data from all completed tests could be used for analysis.

Alternative methods of collecting hand dexterity data using a rotational design instead of magnetic other sensors could include the use of a motion control sensor or rotation sensor, such as a rotary encoder (absolute or incremental). Such a sensor converts rotational mechanical displacement into an electrical signal from which outputs such as speed and position are determined. Other types of rotational sensors that could be used to similar effect include hall effect sensors, potentiometers, optical encoders or rotary variable differential transformers (RVDT). This category of sensors also includes rotational speed sensors. These use various magnetic proximity principles to detect the speed of components. This family includes differential hall sensors, magneto inductive sensors, inductive sensors, oscillatory sensors, eddy current sensors or proximity switches. An infra-red (IR) sensor may also be used.

Hand dexterity is an important outcome measure in most neurological conditions as loss of motor function is a common symptom in these conditions. This includes but is not limited to Parkinson's Disease, Multiple Sclerosis, Stroke, Cervical Myelopathy or other rarer neurological that affect hand function.

## Claims

1. A device for measuring finger dexterity, comprising
a thumb portion configured to be releasably secured to a thumb,
a finger portion configured to be releasably secured to an index finger, wherein the finger portion and the thumb portion are connected by a flexible connector;
a first rotatable element;
a rotation sensor configured to sense rotation of the rotatable element,
a microprocessor, wherein the microprocessor is in communication with the rotation sensor, and
a transmitter configured for wireless communication with a computing device,
wherein the rotatable element is configured to rotate when the finger portion and thumb portion move relative to each other, and wherein the microprocessor is configured to generate rotation data in response to rotation of the rotatable element, wherein the transmitter is configured to transmit the rotation data to the computing device.

2. The device of claim 1, wherein the rotatable element is a magnet and wherein the rotation sensor is a magnetic rotary encoder.

3. The device of claim 1, wherein one end of the flexible connector is affixed to the finger portion and the other end of the flexible connector is affixed to the thumb portion, and wherein the flexible connector is configured to wind around a second rotatable element when the thumb portion and finger portion move towards each other.

4. The device of claim 3, further comprising a spiral torsion spring fixed to the second rotatable element, wherein the spiral torsion spring is arranged to contract when the thumb portion and the finger portion are moved away from each other.

5. The device of claim 4, wherein contraction of the spiral torsion spring causes rotation of the magnet.

6. The device of any preceding claim, wherein the microprocessor and transmitter are housed in a wrist portion, wherein the wrist portion comprises securing means for securing the wrist portion to a wrist.

7. The device of any preceding claim, further comprising a timer configured to record the time required to complete a predetermined number of finger taps by a participant performing a finger tapping test.

8. A system comprising the device of any of claims 1 to 7 and a computing device configured to receive time and magnet rotation data, plot a graph of magnet rotation data as a function of time and output the graph on a display of the computing device.

9. The system of claim 8, wherein the computing device is configured to convert the magnet rotation data into distance data by division by a predetermined calibration factor.

10. The system of claim 8 or 9, wherein the computing device is further configured to store data relating to the maximum finger extension of a participant.

11. The system of claim 10, wherein the computing device is further configured to calculate a score for each finger tapping test performed, wherein the score is a normalised height value divided by a normalised time value.

12. The system of claim 11, wherein the normalised height is an average maximum extension distance between thumb and index finger recorded during a test divided by a maximum possible extension between thumb and index finger for a particular participant.

13. A method of measuring finger dexterity, comprising
generating, by a device according to any one of claims 1 to 8, time and magnet rotation data pertaining to a finger tapping test,
wirelessly transmitting the time and magnet rotation data to a computing device,
generating, by the computing device, a graph of magnet rotation data as a function of time and
outputting, by the computing device, the graph on a display.

14. The method of claim 13, further comprising calculating, by the computing device, a score value for a finger tapping test using standardised data.

15. The method of claim 13 or claim 14, further comprising analysing the data, by the computing device, to determine at least one of average extension height, maximum extension height, time taken to complete a predetermined number of finger taps, number of hesitations and time taken to complete one tap motion.
